# EUROPEAN PATENT SPECIFICATION

(11) **EP 1 684 823 B1**
(45) Date of publication and mention of the grant of the patent: **10.04.2013**
(21) Application number: 04796398.8
(22) Date of filing: 26.10.2004
(51) Int. Cl.: A61M 5/178, A61M 25/00, A61M 25/06

(54) **SAFETY SPINAL CATHETER**
SICHERHEITSWIRBELSÄULENKATHETER
CATHETER RACHIDIEN SUR

(30) Priority: 27.10.2003 US 694235
(43) Date of publication of application: 02.08.2006
(73) Proprietor: Custom Medical Applications, Inc., Johnstown, NY 12095 (US)
(72) Inventor: RACZ, N., Sandor, Irving, TX 75038 (US); WILEY, Christopher, Hanover, NH 03755 (US)
(74) Representative: Bond, Laurence Blair
(86) International application number: PCT/US2004/035411
(87) International publication number: WO 2005/044335

(56) References cited:
- US-A- 5 116 323
- US-A- 5 370 624
- US-A- 5 871 470
- US-B1- 6 245 044
- US-B2- 6 558 353

## Description

### TECHNICAL FIELD

This invention relates to medical catheters. It is particularly directed to spinal catheters.

### BACKGROUND:

The advantages of continuous spinal anesthesia have long been appreciated by anesthesiologists. Unlike conventional single-shot techniques, continuous spinal anesthesia ("CSA") with an indwelling catheter allows anesthesia of unlimited duration and the ability to carefully control the level of the block by repeated small incremental doses of anesthetic. As compared to continuous epidural anesthesia, which has become widely used as a substitute for spinal, CSA generally requires far less drug to achieve the desired effect, has a definite endpoint of correct catheter placement, requires no "test dose," and produces a much more reliable, less spotty block.

Unfortunately, technical problems have severely limited the usefulness of continuous spinal techniques. Until recently, the standard technique of inserting the spinal catheter through the spinal needle, coupled with the difficulty of manufacturing truly small needles and catheters, has meant large needles and catheters were required, resulting in an unacceptably high incidence of post-dural puncture headache ("PDPH").

In the mid 1980's, various advances fueled renewed interest in spinal anesthesia in general and in CSA in particular. Improvements in manufacturing ever-smaller conventional (Quincke™) spinal needles of 25g, 26g, and even 30g significantly reduced PDPH incidence. These results allowed for the use of spinal anesthesia in age groups and procedures not previously considered suitable.

At the same time, advances in catheter manufacture made possible spinal catheters of 28g and 32g which would fit through relatively small spinal needles. Unfortunately, these catheters proved difficult to handle, difficult to make, very expensive, and more ominously, associated with several reports of neurologic damage (*i.e*., cauda equina syndrome). Many clinicians therefore tried and abandoned them, and they were ultimately removed from the market by the Food and Drug Administration ("FDA").

A parallel technical development has been the introduction of non-cutting spinal needles, such as the "Pencil Point" type needles, which have been shown to drastically reduce PDPH incidence. Examples of Pencil Point type needles include the Sprotte and Whitacre non-cutting spinal needles. In terms of PDPH incidence, a 22g Sprotte seems to be roughly equivalent to a 25g or 26g Quincke, while a 24g Sprotte or 25g Whitacre essentially eliminates the risk of PDPH.

The FDA's decision to recall and ban the marketing of microspinal catheters for CSA in the U.S., and its requirement that any new device for CSA be subjected to an extremely stringent pre-market approval process, has resulted in a complete freeze on the development of these products, at least in the United States. Nevertheless, the injection of local anesthetics for the establishment of surgical anesthesia is not the only use to which such devices might profitably be put. In fact, the injection of narcotics, such as FENTANYL®, for analgesia of labor would be a very desirable use of such catheters.

Installing a conventional catheter generally requires various cumbersome steps involving threading long, very thin catheters through a spinal needle. Simply threading a catheter into the end of a spinal needle can be so difficult that some manufacturers include a "threading aid" as part of their kit. Once threaded, a degree of uncertainty exists for the clinician about how far to insert the catheter. Also, a risk exists that a piece of the catheter might be sheared off by the needle if the catheter were to be pulled back during the threading operation. In such case, bits of catheter could potentially be left behind in the intrathecal space. Furthermore, removing the spinal needle while holding the catheter in position can be a challenge. Additionally, attaching a hub/injection adapter to the naked end of the 28g or 32g catheter can be even more of a challenge. Finally, once the adapter is successfully attached, the small lumen of the catheter permits only a slow flow of either CSF or anesthetic. In short, the conventional spinal catheter threading operation requires considerable time and effort on the part of a clinician.

One problem of Sprotte and Whitacre non-cutting spinal needles is that the injection orifice is on the side of the needle. Failures of spinal anesthesia have been described as when the needle was "half-in, half-out" of the intrathecal space. Another problem with Sprotte and Whitacre spinal needles is that the smooth curved tip profile provides no definitive feedback signal or "click" when the dura is punctured. Such lack of feedback contributes to uncertainty of catheter tip placement.

Conventional spinal catheters are very long and thin. As such, they are relatively cumbersome to handle without accidental contamination. They also can be difficult to secure to the skin, and can be prone to kinking at the skin or to inadvertent removal by patient movement. This kinking can result in damage to the catheter. Moreover, reports have been made of neurologic damage associated with micro-catheters. Thus, CSA itself has been abandoned in the United States, although it remains popular in Europe.

One example of a spinal needle set is disclosed in U.S. Patent 5,871,470, issued February 16, 1999, to Mc Wha, Briefly, the spinal needle set includes an epidural needle, a spinal needle disposed within a bore of the epidural needle, and an optional stylet disposed within the spinal needle. An open distal end of the epidural needle is beveled for insertion through the skin of a patient into the epidural space. The "pencil point" distal end of the spinal needle is then advanced from within the bore of the epidural needle, through the dural membrane, into the subarachnoid space, Both the epidural needle and the spinal needle are preferably formed from stainless steel,

### DISCLOSURE OF INVENTION

In contrast to a conventional spinal catheter, the instant invention provides for simple and straightforward catheter insertion without either threading a catheter through a needle or installing an adapter. The installation procedure is similar to intravenous catheter or single-shot spinal procedures already familiar to clinicians. Placement of the catheter over the inserting needle allows a larger diameter catheter to be inserted. The resulting improved diameter catheter allows easier and faster flow of either CSF or medicating agents.

Insertion of the catheter tip in the intrathecal space with the instant device is more secure. The Pencil Point style non-cutting tip of the support needle promotes a low incidence of PDPH. However, the assembly tip may be shaped to provide a feedback signal when the dura is punctured. Observation of CSF with the instant design further assures a clinician that the entire orifice at the catheter tip is in the intrathecal space.

The chance of neurologic damage is lessened with the shorter catheter of the present invention. The shorter length is less likely to be wedged against a nerve root. More importantly, the larger bore of the improved catheter promotes turbulent flow and improved mixing of any injected fluid will occur with CSF. The improved short catheter, which is inserted to the hub, removes ambiguity about how far to insert. The catheter hub greatly aids fixation to the skin. Contamination during insertion is less likely. Also, kinking at the skin is essentially impossible when a flexible kink sleeve is included.

The ease, simplicity, and relative safety of the improved device may expand the use of continuous spinal anesthesia/analgesia. Essentially all lumbar epidurals could be replaced with this apparatus. Similarly, most single-shot spinals may be replaced with this apparatus "just-in-case" the procedure goes longer than expected, or the level of the block needs adjustment. A number of situations outside the operating environment could benefit from this device, non-exclusively including: acute and chronic pain control with spinal narcotics, labor analgesia, diagnostic taps, and indwelling catheters for continuous peripheral nerve blocks as well as research purposes. In effect, this apparatus can be used in every medical procedure involving needle insertion at the lumbar level of the spine. Versions of the instant device are contemplated to offer improved techniques for the insertion of a wide variety of medical catheters, including arterial lines, major nerve blocks, intraperitoneal catheters, intraventricular (brain) catheters, and intravenous catheters.

The present invention provides an apparatus and method for inserting a spinal catheter in a quick, easy, and straightforward manner. Such a spinal catheter assembly has an outside diameter sized so that withdrawal of the assembly from the subarachnoid space, subsequent to insertion of the assembly thereby, permits the dura mater substantially to reseal a space formerly occupied by the assembly. An assembly typically includes a support needle, a catheter slidably mounted on the support needle, and a central stylet slidably inserted within the support needle. The inserted tip end of a catheter assembly is advantageously configured to produce a feedback signal to indicate dural puncture.

A support needle preferably has a piercing point on a first end and a central hub at a second end. The piercing point protrudes from a front, distal, inserted, or tip, end of a catheter assembly. A piercing point penetrates substantially without cutting, and helps to form a puncture hole through dura mater which automatically may substantially reseal subsequent to retraction of a catheter. A second end of the central stylet generally has a locking hub. The locking hub may carry a first attach structure to connect with corresponding structure of a central stylet.

The front end of the support needle may be configured cooperatively to form a structural interference with a distal end of a catheter. Such structural interference resists relative motion between the piercing point and the distal end of the catheter during insertion of the catheter into a patient. A rear end of the support needle may carry a support hub having second attach structure to removably connect to the central hub of the central stylet. The first and second attach structures maybe structured to form a removable connection, such as a LUER-LOCK^{®} type connection. The support hub is advantageously made from a transparent material to permit observation of fluid flow therethrough.

A catheter may be characterized as a flexible conduit having distal and proximal ends. Preferred catheters have sufficient transverse flexibility to accommodate patient torso bending movement, whereby substantially to reduce a catheterized patient's awareness of the presence of the catheter. Catheters typically are made from medical grade plastic materials. For example, polyester shrink tube or similar materials may be used. The distal end of a catheter may be reinforced, in some instances, to resist peel-back from the front end of a support stylet. Such reinforcement may be by way of tip forming or wrappings of fine gauge wire or by a safety ribbon band. The wire or band may be made from any suitable structurally reinforcing material, including stainless steel. The proximal end of a catheter generally carries a catheter hub having a third attach structure. This third attach structure may be adapted to structurally interfere in a releasable locking arrangement with a structure carried by the support needle.

The transition from the proximal catheter hub to the catheter body may be reinforced by a kink sleeve segment. The kink sleeve segment may be constructed of a firm yet flexible material, such as a nylon or other polymer. The kink sleeve is intended to cushion the transition from the hub to the catheter body during bending that will occur after the catheter is inserted and the support needle removed. For example, once the catheter is inserted, the hub may be bent over and taped to the skin, often at an angle of around 90 degrees.

Catheter hubs are typically configured for fluid flow attachment to medical fluid transfer equipment. For example, catheter hubs may be configured to form LUER-LOCK^{®} type connections with such equipment. It may be further preferred to form the catheter hub for substantially unobtrusive attachment to a patient's skin by way of an intermediary adhesive element or by designing the hub to lay flush against the patient's skin with a connection parallel thereto without a need for bending the catheter.

A spinal catheter assembly may be installed using a method similar to the following: providing a spinal catheter assembly according to this invention; using conventional spinal needle technique to prepare skin of a patient at an injection site, apply local anesthetic, pierce skin and subcutaneous fascia, and insert a piercing point tip of the catheter assembly; removing the central stylet subsequent to receiving a feedback signal that puncture of the dura mater has occurred; checking for CSF at the support hub; if no CSF is observed, further inserting the assembly until the tip is within the intrathecal space; or if CSF is observed, unlocking the support hub and the catheter hub, and while holding the support needle stationary, advancing the catheter until the catheter hub contacts the skin; removing the support needle and checking for the presence of CSF at the catheter hub; connecting medical fluid transfer apparatus to the catheter hub; and finally, securing the catheter hub to the skin.

These features, advantages, and additional alternative aspects of the present invention will be apparent to those skilled in the art from a consideration of the following detailed description taken in combination with the accompanying drawings.

### BRIEF DESCRIPTION OF DRAWINGS

In the drawings, which illustrate what is currently regarded as the best mode for carrying out the invention and in which like reference numerals refer to like parts in different views or embodiments:
FIG. 1 is an exploded plan assembly view of one embodiment of a catheter assembly according to the present invention.
FIG. 2 is a cross sectional view of a portion of a catheter hub in accordance with one embodiment of the present invention.
FIG. 3 is a plan view of the assembled catheter assembly in FIG. 1.
FIGS. 3A to 3H are plan views of different aspects of interactions between a catheter hub and a support hub, in accordance with the present invention.
FIG. 4 is a cutaway plan view of another embodiment of a catheter hub in accordance with the present invention.
FIG. 5 is a detail view of the distal end portion of the catheter assembly tip, circled and designated by numeral 4, in FIG. 3.

### BEST MODE FOR CARRYING OUT THE INVENTION

The present invention may be constructed as an integrated spinal needle and catheter assembly 10 (much like an intravenous needle and catheter) in which the catheter 15 is on the outside. Placement of the catheter 15 on the outside provides a number of advantages. First, this design makes insertion significantly easier by eliminating the separate steps of catheter threading, insertion and hub/adapter attachment. A single "stick" is all that is required; once the needle is in, so is the catheter. Since the catheter 15 is larger for a given needle size, its flow and handling characteristics will be much improved, and it is easier and cheaper to manufacture.

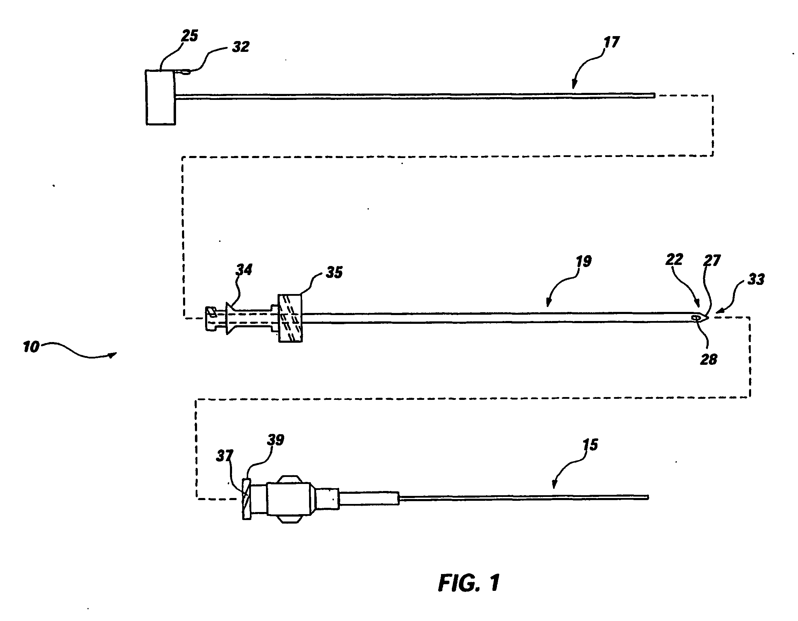

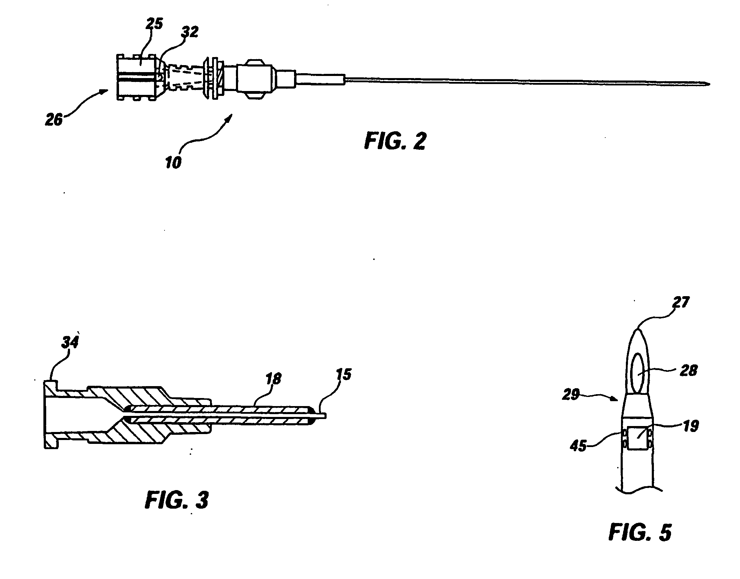

Embodiments of one possible catheter assembly 10, in accordance with the principles of the present invention, are illustrated in FIGS. 1 & 2. The illustrated assembly 10 consists of three components: a central stylet 17, a hollow support needle 19, and a catheter 15. The overall dimensions of the currently preferred embodiment of the assembly 10 are similar to a conventional spinal needle of between 22g and 24g.

The innermost component of the assembly is preferably fashioned as a solid central stylet 17. When inserted in the support needle 19 (discussed in detail further herein), the central stylet 17 prevents the entry of extraneous tissue or other material into the support needle opening 28 during insertion. The central stylet may also serve as a "stiffening" portion of the assembly providing extra support and stiffness to the entire assembly. The hub 25 of the central stylet 17 is outermost, or located at an extreme proximal end 26 of assembly 10, because the central stylet 17 is the first to be removed. An attachment structure, such as tab 34, may be located on the hub 25 for retaining the central stylet 17 in the support needle 19. The tab 34 may interact with a corresponding attachment structure on the hub 35 of the support needle 19.

The next layer of the assembly is a removable hollow support needle 19 to support and allow insertion of the catheter 15. This support needle 19 closely resembles a conventional spinal needle. The tip 27 of support needle 19 may have a pencil-point formation to allow penetration of tissue substantially without cutting. As discussed previously herein, this aids in forming a puncture hole through the dura mater which automatically may substantially reseal subsequent to retraction. An opening 28 is located near the tip 27 to allow CSF or other fluids to flow through the support needle 19 from the opening 28 to the hub 35. It will be appreciated that where desired, suitable treatment solutions may be inj ected through the support needle 19, to enter a patient's tissue through the opening 28.

The hub 35 of the support needle 19 may beneficially be made of clear plastic to allow visualization of CSF return when the central stylet 17 has been removed. Of course, any present CSF will visibly flow from the distal end 3 3 of support needle 19 subsequent to removal of the central stylet 17. Optional use of clear plastic or a transparent fluid observation window in the support hub 35 can provide an additional convenience, and minimize loss of CSF.

The central stylet 17 may be attachable to the support needle 19, as illustrated in FIGS. 1 & 2. The central hub 25 typically carries an attach structure, such as tab 32, to interface in a structural interference with an attach structure 34 carried by support hub 35. As illustrated, tab 32 and attach structure 34 cooperatively form a slidably engageable joint. Alternative releasable retaining joint configurations, including rotatable attachments such as LUER-LOCK^{®} type joints, may also be used.

The outermost layer of the assembly 10 is the catheter 15 itself. It preferably is approximately 23g and about the length of a conventional spinal needle, although different diameters and lengths for use with different procedures is within the scope of the present invention. Conventional plastic catheter material may be used in its construction. The catheter material may be reinforced with a flat ribbon internal spring 45 (shown in FIG. 5), an internal or external wire wrap, or other reinforcing structure. Alternative materials, and various materials in combination, also may be used to construct a catheter 15. Suitable catheter material produces a catheter 15 which is fairly stiff and has a sufficiently high tensile strength to maintain structural integrity during insertion, while in the body, and during retraction from a patient. A catheter 15 desirably possesses sufficient transverse flexibility to deform and accommodate patient motion to reduce irritation from the presence of a foreign body.

A slippery nonstick surface is generally provided to ease insertion and removal of the catheter 15. The tip 29 of catheter 15 is tapered into a curve to blend smoothly into the edge of support needle 19 (*see,* FIG. 5). The degree of this curved taper may be governed by a tradeoff between the decreased resistance to insertion of an extreme taper versus the fragility and tendency to peelback of a very thin leading edge. A preferred taper provides ease of insertion, a feedback signal to indicate entry of catheter 15 through the dura, and sufficient tensile strength to prevent peelback. The feedback signal may be described as a distinct "click" or a change in required insertion force. The "click" may be a sonic event, or may be perceptible only through the clinician's fingers in contact with the assembly.

Catheter tips 29 having shapes other than those illustrated in FIGS. 1, 3 & 5 are within contemplation. For example, manufacturing or material requirements may influence the shape of a tip 29. An alternative catheter may include a reinforcing wire of fine gauge. Such a wire may be embedded into the material forming the wall of catheter 15 to reinforce against peelback. The wire may also be spiraled along the length of the catheter to provide additional strength to resist collapse, kinking, or breakage of a catheter 15. Alternatively, a flat spring ribbon 45 may be used to provide reinforcement.

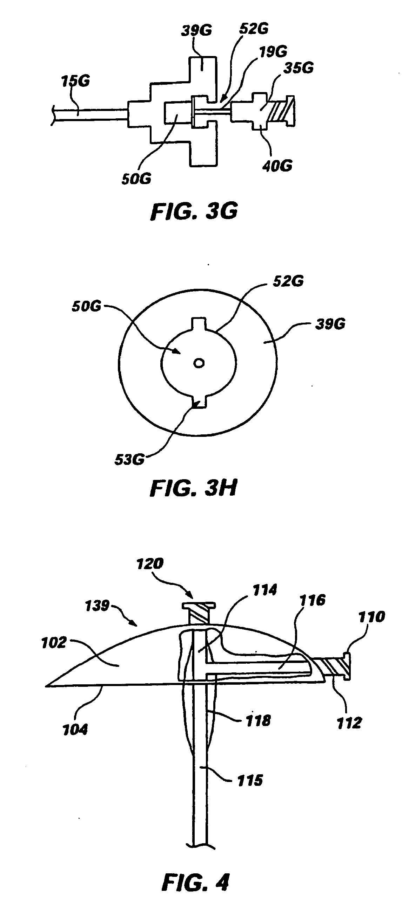

The catheter hub 39 typically includes a LUER-LOCK^{®} type connector, or other attachment structure, for easy and secure connection with common infusion tubing, injection ports, or syringes, and other medical fluid transfer apparatus. Since the catheter 15 may be inserted all the way to the hub 39, a flat, circular flange, or other ergonomically shaped structure, may be provided on the surface of the hub which rests against the patient's skin to facilitate easy tape fixation. Fixation to the patient's skin may be accomplished with a slotted circular foam tape. Of course, other tapes or adhesive systems may also be used. A quantity of suitable adhesive or tape could be included in a prepackaged catheter kit. A catheter hub 139 (see FIG. 4) that lays flat against patient's skin and allows attachment of a line at an angle substantially parallel to the skin, rather than generally perpendicular thereto, may be used. Such a catheter hub may be more comfortable for a lengthy procedure.

It is desirable to prevent inadvertent premature removal of the support needle 19 from the catheter 15. In the embodiment depicted in FIGS. 1-3, support hub 35 receives thread structure 37 located on the catheter hub 39 and locks with rotation. Such a positive connection may be desirable and can form a LUER-LOCK^{®} or other rotatable-type joint. Other such interlocking or even alternative retaining structure may also be used. For example, a secure friction fit attachment between support needle 19 and catheter 15 is within contemplation in the practice of this invention, as is a structural interference fit of attachment structures similar to shown in connection with tab 32 on the central stylet 17.

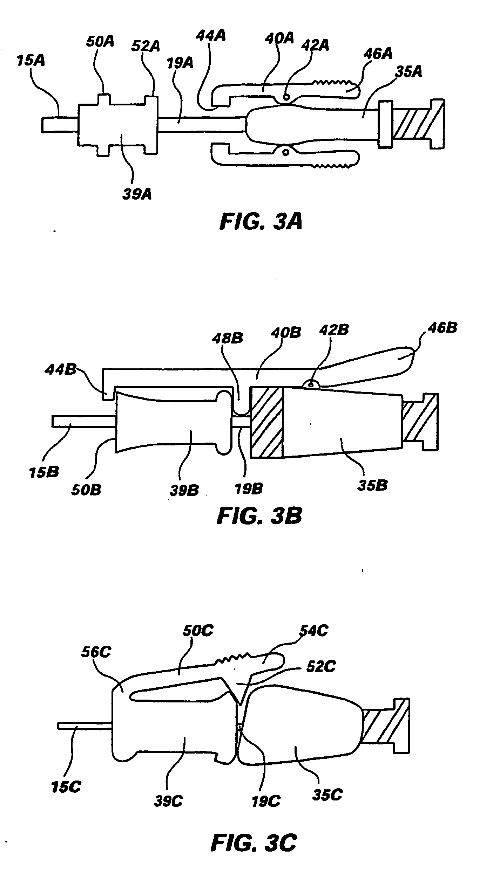

FIGS. 3A to 3G depict several aspects of relationships between the hubs of a catheter 15 and a support needle 19 that may prevent premature removal of the support needle 19 or aid in support needle 19 removal at the appropriate point of a procedure. FIG. 3A depicts a support needle 19A having a support hub 35A that includes a plurality of retaining levers 40A. Each lever 40A is attached to the body of the support hub 35A by a pivot structure, such as pin hinge 42A, allowing the distal end of the retaining lever 40A to be rotated away from hub 35A as the proximal end 46A is depressed. The retaining levers 40A expand around the catheter hub 39A allowing the catheter hub 39A and support hub 35A to be slid together as the needle 19A is inserted in the catheter 15A. The distal end of the retaining levers 40A include an attach structure, such as the enlarged end 44A, that interacts with a corresponding attach structure, such as the ridges 50A and 52A on the catheter hub 39A, to retain the hubs in position to one another. Ridges 50A and 52A may be formed as discrete bumps located on the catheter hub 39A or may be formed as raised ridges running around the entire circumference of the hub 39A. To release support needle 19A from catheter 15A, retaining levers 40A are depressed at the proximal end 46A, which may include a grip structure, and the hubs may then be separated. It will be appreciated that although two retaining levers 40A are depicted, any suitable number may be used and all such embodiments are within the scope of the present invention.

FIG. 3B depicts a somewhat similar arrangement where a retaining lever 40B is rotatably attached to the support hub 35B and includes an enlarged distal end 44B with a lip that forms a structural interference fit with an attach structure, such as distal end 50B of the catheter hub 39B. Additionally, retaining lever 40B includes a detach assisting structure, such as detach bar 48B that resides between the catheter hub 39B and the support hub 35B in the retained position. When proximal end 46B of the retaining lever 40B is depressed, distal end 44B rotates out from the catheter hub 39A releasing it. Simultaneously, detach bar 48B presses against the proximal end of the catheter hub 39B, causing support needle 19B to begin withdrawing from catheter 15B. It will be appreciated that although only one retaining lever 40B is shown for clarity, any desired number of retaining levers 40B may be used. Further, although a pin hinge 42B is depicted, any suitable rotatable connection, such as a living hinge formed from injection molded plastic, may be used.

FIG. 3C depicts another detach assisting structure for removing support needle 19C (not shown) from catheter 15C. In such an embodiment, the catheter hub 39C and support hub 35C may be releasably attached to one another by a friction fit, or by a small amount of a weak adhesive. A detach lever 50C is attached to the catheter hub 39C and includes a detach assisting structure such as detach wedge 52C. Detach lever 50C maybe attached to the catheter hub 39C in any suitable fashion, as by a pin hinge or by forming a living hinge 56C by fashioning detach lever 50C as an extension of the distal end of the catheter hub 39C. The detach wedge 52C may be disposed so the leading edge thereof is disposed between the distal end of the support hub 35C and the proximal end of the catheter hub 39C body. To actuate detachment, the detaching lever 50C is depressed by pressing on the proximal end 54C thereof causing he detachment wedge 52C to advance further between the hub 35C and 39C forcing them apart and the support needle 19C to withdraw from the catheter 15C.

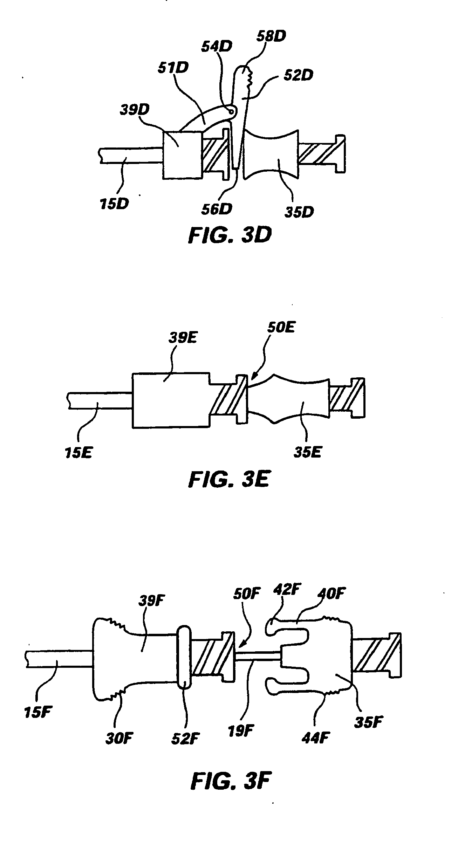

Another example of aspects of a detach assisting structure is depicted in FIG. 3D. A detach lever 52D is rotatably attached to catheter hub 39D through a pin hinge 54D or another flexible connection. The attachment may occur on a protrusion, or detachment extension 51D, extending out from the catheter hub 39D body. Detach lever 52D has a detaching end 56D that resides between the catheter hub 39D and the support hub 35D when the hubs are in the retained position. The opposite actuation end 58D of the detach lever 52D may include a grip area formed as a roughened surface. Detach lever 52D is actuated by pressing the actuation end 58D in the distal direction causing the detaching end 56D to rotate out from the catheter hub 39D pressing against the distal end of the support hub 35D, causing support needle 19D (not shown) to begin withdrawing from catheter 15D.

FIG. 3E depicts another aspect of a connection between a catheter hub 39E and a support hub 35E. Catheter hub 39E has an enlarged bore opening 50E at its proximal end into which the distal end of support hub 35E may be inserted upon insertion of the needle 19E (not shown) into catheter 15E. The walls of enlarged bore opening 50E and the distal end of support hub 39E fit together snugly forming a friction fit there between to retain the hubs together. The hubs may be constructed of material selected for a suitable coefficient of friction to maintain the relationship between the hubs.

FIG. 3F depicts a catheter hub 39F including an enlarged bore opening 50F and a support hub 35F having a relationship similar to that described with respect to FIG. 3E. Additional retention structures are also depicted. Support hub 35F includes a lip 40F extending distally from the hub body to create a recess 41F. Lip 40F includes an enlarged distal end 42F and may be resilient As the needle 19F is inserted into catheter 15F and a portion of the support hub 35F is inserted into enlarged bore opening 50F, lip 40F passes over a portion of the catheter hub 39F, flexing outward to allow enlarged distal end 42F to pass over a ridge 52F on the catheter hub 39F. Enlarged distal end 42F blockably interacts with ridge 52F to prevent inadvertent removal of the needle 19F. At the appropriate point in the procedure, the hubs may be separated by applying sufficient force to the hubs in opposite directions to cause the lip 40F to flex and allow the enlarged distal end 42F to pass over the ridge 52F. Grip points 50F and 44F may be provided on the catheter hub 39F and support hub 35F, respectively, to assist in the removal of the needle 19F. It will be appreciated that lip 40F may be formed as an extension around the entire circumference of the support hub 35F taking the shape thereof, whether generally circular or otherwise, or may be formed as a plurality of separate extensions, and all such embodiments are within the scope of the present invention.

FIGS. 3G and 3H depict a rotatable retaining relationship between catheter hub 39G and support hub 35G. Support hub 35G includes a number of discrete protrusions, such as retaining tabs 40G at a point along the hub body. Catheter hub 39G includes an enlarged bore opening 50G into which a portion of the body of the support hub 35G my be inserted. The mouth 52G of enlarged bore opening 50G is best depicted in FIG. 3H. A central section of mouth 52G allows the support hub 35G body to pass therethrough, yet is too small to allow the tabs 40G to similarly pass. Mouth 52G includes bays 53G extending into the proximal end of the catheter hub 39G from the central section of the mouth 52G. Each bay 53G corresponds to a tab 40G and allows passage therethrough to the enlarged bore opening.

When support hub 39G is fully inserted into the enlarged bore opening 50G, support needle 19G is fully inserted in the catheter 15G and tabs 40G reside in the enlarged bore opening 50G. Support hub 35G may then be rotated with respect to the catheter hub 39G, so that tabs 40G no longer align with bays 53G. Support needle 19G is then blockably prevented from premature removal from the catheter 15G. Removal can be accomplished by rotating the support hub 39G to align tabs 40G with bays 53G and then slidably withdrawing the support hub 35G. It will be appreciated that although two tabs 40G and bays 53G are depicted, any suitable number may be used.

As best shown in FIG. 3, catheter 15 may include a flexible kink sleeve 18. Kink sleeve 18 covers a portion of the proximal surface of the catheter 15 to protect the area covered against kinking and damage during bending. Desirably, the kink sleeve 18 will begin at the base of the catheter 15 inside the hub 39 (as depicted in FIG. 3) to provide maximum protection, although alternate embodiments where kink sleeve begins distal to the base of the catheter inside the hub 39, or at the base of the hub 39 are within the scope of the present invention. Kink sleeve 18 may extend distally along the length of the catheter 15 to a length appropriate for the planned use of the catheter. Typically, kink sleeve 18 will extend to a length sufficient to prevent kinking of the catheter at the skin of the patient or within the skin and fascia of the patient. Kink sleeve 18 may be constructed of any suitable flexible material that is medically acceptable, including polymers such as nylon.

When catheter 15 is fully inserted, a portion of the kink sleeve 18 will reside within the skin and fascia of the patient. The hub 39 may then be bent over and taped to the skin, if desired. The kink sleeve 18 acts to protect the catheter 15 during this bending process, which may bend the catheter 15 at an angle of about 90 degrees or more. The kink sleeve 18 absorbs the force of the bend and maintains the catheter 15 in a position allowing flow therethrough. Kinking of the catheter 15 is thus minimized, and may be prevented. The kink sleeve 18 may be impregnated, coated, or otherwise treated with a biocompatible infection resistant substance to prevent adverse tissue reaction or infection at the catheter entry site. Embodiments where the catheter hub 139 (FIG. 4) lies flat against the skin, allowing attachment at an angle generally perpendicular to insertion may further avoid potential kinking. Similar to kink sleeve 18, the catheter hub 139 may be impregnated, coated, or otherwise treated with a biocompatible infection resistant substance to prevent adverse tissue reaction or infection at the catheter entry site.

FIG. 4 depicts a catheter hub 139 that lies flat against the patient's skin and allows a line to be attached at an angle generally perpendicular to the direction of catheter insertion. The hub 139 includes a body 102 with a generally planar proximal end 104, from which the catheter 115 extends. A kink sleeve 118 may be included on the catheter 115. A bore 114 continuous with the bore of the catheter 115 extends in line therewith through the body 102 to allow placement of the support needle 19 and central stylet 17 through alignment opening 120. A connection bore 116 connects to the bore 114, from an angle generally perpendicular thereto and passes to a connection outlet 110 generally perpendicular to the angle of insertion of catheter 115. The junction between connection bore 116 and bore 114 may be formed as a T-shaped junction as depicted in FIG. 4.

Connection outlet 110 may include a connection structure, such as the LUER-LOCK^{®} type threads 112 depicted in FIG. 4, in order to allow tubing, a connection line, a syringe or other structure to be attached thereto in communication with connection bore 116 and bore 114. A line connected to connection outlet 110 may lay flat on the skin of a patient resulting in a more comfortable connection than a perpendicular connection.

Similarly, alignment opening 120 may include a connection structure, such as LUER-LOCK^{®} type threads, in order to allow tubing, a connection line, a syringe or other structure to be attached thereto in communication with bore 114. Upon withdrawal of the support needle 19 after catheter 15 placement, alignment opening 120 may be closed by capping, with a cap or an injectable port (to provide another point for the introduction of suitable treatment solutions to the catheter 15). In some embodiments, a resealable puncturable membrane may be provided across the alignment opening 120 (or the bore 114 above connection bore 116) to allow insertion of a support needle and central stylet therethrough, while sealing the bore 114 upon their removal.

Catheters 15 may be made from suitable medical grade plastic type materials. For example, polyester shrink tubing may be employed with one embodiment of the device, although it will be appreciated that any suitable material, including other polymers, maybe used. Catheters 15 may be composed of a single material, or may be a composite of two or more materials to provide the desired catheter handling characteristics. Fine gauge wire, such as stainless steel wire, or a flat internal ribbon spring 45, may be incorporated into a catheter wall to improve resistance to peelback. The distal ends may alternatively be reinforced with metal bands. Hubs 25, 35 and 39 are typically also made from medical grade plastic type materials. The central stylet 17 and support needle 19, are typically made from a medically acceptable metal, such as stainless steel or titanium.

The design of this device makes the placement of a spinal catheter 15 quick, easy, and straightforward. It should be so easy, in fact, that most clinicians may choose to use this device for every spinal procedure they perform. The initial steps of skin preparation, local anesthetic infiltration, and needle insertion are identical to those now used with conventional spinal needles. As the assembly 10 is being inserted and the clinician feels the slight "click" upon dural puncture, he or she removes the central stylet 17. If the insertion has been successful, CSF will promptly appear at the hub 35 of the support needle 19. If the dura has not been penetrated, the entire assembly 10 may continue to be advanced until dural puncture is achieved. If desired, the central stylet 17 may be reinserted prior to continued advancement in order to prevent tissue from entering the opening 28.

Once CSF is observed at the hub 35 of the support needle 19, the clinician can be certain that the tip 29 of the catheter 15 is within the intrathecal space. If desirable for the procedure, the clinician may continue to advance the hollow needle/catheter 19/15 assembly another centimeter or so. At this point, the hub 35 of the hollow needle 19 is typically twisted to unlock it from the catheter hub 39 or 139, and while holding the hollow needle 19 stationary, the catheter 15 is advanced all the way until the hub 39 or 139 contacts the patient's skin. For embodiments including a kink sleeve 18, this advancement inserts, or further inserts, the kink sleeve 18 within the patient's skin.

At this point, the hollow support needle 19 may be removed, and the appearance of CSF at the catheter hub 39 or 139 will confirm the correct placement of the catheter 13. The desired injection port, tubing, or other medical fluid transfer apparatus, may then be attached to the catheter hub 39 (or 139) such as by way of attach structure 37 (or 112). Where necessary, the catheter 15 may be bent and taped to the patient's skin before or after the attachment of the corresponding apparatus, if required. Where included, kink sleeve 18 protects the catheter 15 from kinking and damage at the bend. A piece of slotted, circular foam tape (which might also be treated with an antimicrobial) may also be applied to fix the hub 39 or 139 to the skin, prevent dislodging of the catheter 15, and cushion the patient to reduce potential irritation from the hub 39 or 139.

The catheter 15 may then be left in place for as long as clinically necessary and, assuming adequate tensile strength, be easily and safely removed when appropriate. At the time of removal, since the non-cutting point 22 of the support needle 19 never lacerated any fibers in the dural membrane, the mesh-like fibers may relax to their original position, thus automatically closing the dural puncture. Therefore the PDPH incidence is expected to be in agreement with Sprotte and Whitacre needles, despite the luxury of a reasonably large catheter 15 in a device according to the instant invention.

## Claims

1. A spinal catheter assembly (10) for insertion through a dura mater into a spine of a patient, said spinal catheter assembly (10) comprising:
a support needle (19) having a first end (33) defining a non-cutting piercing point (22), said support needle (19) further comprising a hollow bore with an opening (28) proximate said first end (33) allowing access to said bore; and
a spinal catheter (15) of a plastic material slidably mounted on a portion of said support needle (19), the support needle (19) adapted to extend through a bore of the spinal catheter (15), such that said first end (33) of said support needle (19) protrudes from said spinal catheter (15) exposing said non-cutting piercing point (22) and said opening (28), wherein a tip (29) of said spinal catheter (15) is tapered into a curve to blend smoothly into the surface of the support needle (19) when the support needle (19) is disposed within the spinal catheter (15).

2. The spinal catheter assembly (10) of claim 1, wherein said non-cutting piercing point (22) comprises a pencil-point tip (27).

3. The spinal catheter assembly (10) of claim 1, wherein said catheter assembly (10) has a tip (27) configured and arranged to provide a feedback signal to indicate dural puncture.

4. The spinal catheter assembly (10) of claim 1, wherein:
a rear end of said support needle (19) carries a support hub (35) having a first attach structure (34); and
a proximal end of said spinal catheter (15) carries a catheter hub (39) having a second attach structure (37) configured to removably attach to the first attach structure (34) carried by said support hub (35).

5. The spinal catheter assembly (10) of claim 4, wherein the first and second attach structures (34) and (37) comprise a LUER-LOCK7 type connection.

6. The spinal catheter assembly (10) of claim 4, wherein said catheter hub (39) is configured for substantially unobtrusive attachment to a patient's skin by way of an intermediary adhesive element.

7. The spinal catheter assembly (10) of claim 4, wherein said catheter hub (39) is configured for attachment to medical fluid transfer equipment by an attachment structure (110) to form a connection generally perpendicular to a direction of catheter insertion.

8. The spinal catheter assembly (10) of claim 1, wherein:
a rear end of said support needle (19) carries a support hub (35); and
a proximal end of said spinal catheter (15) carries a catheter hub (39) having a detach structure (48B), (52C), or (52D) configured to detach the catheter hub (39) from the support hub (35).

9. The spinal catheter assembly (10) of claim 1, wherein:
a proximal end of said spinal catheter (15) carries a catheter hub (39); and
a rear end of said support needle (19) carries a support hub (35) having a detach structure (48B), (52C), or (52D) configured to detach the catheter hub (39) from the support hub (35).

10. The spinal catheter assembly (10) of claim 1, wherein said spinal catheter (15) comprises a conduit formed from the plastic material and radially reinforced at a distal end by a second material to resist peel-back from the support needle (19).

11. The spinal catheter assembly (10) of claim 10, wherein said second material is selected from the group comprising a stainless steel wire and a ribbon spring (45).

12. The spinal catheter assembly (10) of claim 1, wherein said spinal catheter (15) comprises a force absorbing structure to prevent kinking when the spinal catheter (15) is bent.

13. The spinal catheter assembly (10) of claim 12, wherein said force absorbing structure comprises a ribbon spring (45).

14. The spinal catheter assembly (10) of claim 12, wherein said force absorbing structure comprises a kink sleeve (18) disposed on a portion thereof.

15. The spinal catheter assembly (10) of claim 1, further comprising a central stylet (17) slidably mounted in said support needle (19) to prevent the entry of matter through said opening (28) proximate said first end (33).

16. The spinal catheter assembly (10) of claim 1, wherein said support needle (19) is configured to resist relative motion between said tip (29) of said spinal catheter (15) and said non-cutting piercing point (22) during insertion of said spinal catheter assembly (10) into the patient.

17. The spinal catheter assembly of claim 7, wherein the connection of said catheter hub (39) to said medical fluid transfer equipment is a LUER-LOCK7 type connection.

## Patentansprüche

1. Wirbelsäulenkatheteranordnung (10) zum Einführen durch eine Dura Mater in eine Wirbelsäule eines Patienten, wobei die Wirbelsäulenkatheteranordnung (10) Folgendes umfasst:
eine Stütznadel (19), die ein erstes Ende (33) aufweist, das eine nicht schneidende Einstechspitze (22) definiert, wobei die Stütznadel (19) ferner eine Hohlbohrung mit einer Öffnung (28), die einen Zugang zu der Bohrung ermöglicht, in der Nähe des ersten Endes (33) umfasst; und
einen Wirbelsäulenkatheter (15) aus einem Kunststoffmaterial, der verschiebbar auf einem Abschnitt der Stütznadel (19) angebracht ist, wobei die Stütznadel (19) angepasst ist, um durch eine Bohrung des Wirbelsäulenkatheters (15) hindurch zu verlaufen, sodass das erste Ende (33) der Stütznadel (19) aus dem Wirbelsäulenkatheter (15) hervorsteht und die nicht schneidende Einstechspitze (22) und die Öffnung (28) freilegt, wobei sich eine Spitze (29) des Wirbelsäulenkatheters (15) in einer Krümmung verjüngt, um glatt in die Oberfläche der Stütznadel (19) überzugehen, wenn die Stütznadel (19) im Wirbeisäutenkatheter (15) angeordnet ist.

2. Wirbelsäulenkatheteranordnung (10) nach Anspruch 1, wobei die nicht schneidende Einstechspitze (22) eine Pencil-Point-Spitze (27) umfasst.

3. Wirbeisäulenkatheteranordnung (10) nach Anspruch 1, wobei die Katheteranordnung (10) eine Spitze (27) aufweist, die konfiguriert und angeordnet ist, um ein Rückmeldungssignal bereitzustellen, um eine Duralpunktion anzuzeigen.

4. Wirbelsäulenkatheteranordnung (10) nach Anspruch 1, wobei:
ein hinteres Ende der Stütznadel (19) einen Stützhub (35) trägt, der eine erste Befestigungsstruktur (34) aufweist; und
ein proximales Ende des Wirbelsäulenkatheters (15) einen Katheterhub (39) trägt, der eine zweite Befestigungsstruktur (37) aufweist, die konfiguriert ist, um trennbar an der ersten, vom Stützhub (35) getragenen Befestigungsstruktur (34) befestigt zu werden.

5. Wirbelsäulenkatheteranordnung (10) nach Anspruch 4, wobei die erste und zweige Bsfestigungsstruktur (34) und (37) eine LUER-LOCK-7-Verbindung umfassen.

6. Wirbejsäutenkathoteranordnung (10) nach Anspruch 4, wobei der Katheterhub (39) konfiguriert ist, um anhand eines Zwischenhaftelements auf im Wesentlichen unauffällige Weise an der Haut eines Patienten befestigt zu werden.

7. Wirbelsäulenkatheteranordnung (10) nach Anspruch 4, wobei der Katheterhub (39) konfiguriert ist, um über eine Befestigungsstruktur (110) mit medizinischer Fluidtransferausrüstung verbunden zu werden, um eine Verbindung zu bilden, die allgemein senkrecht zu einer Richtung der Kathetereinführung verläuft.

8. Wirbelsäulenkatheteranordnung (10) nach Anspruch 1, wobei:
ein hinteres Ende der Stütznadel (19) einen Stützhub (35) trägt; und
ein proximales Ende des Wirbelsäulenkatheters (15) einen Katheterhub (39) trägt, der eine Trennungsstruktur (48B), (52C) oder (52D) aufweist, die konfiguriert ist, um den Katheterhub (39) vom Stützhub (35) zu trennen.

9. Wirbelsäuienkatheteranordnung (10) nach Anspruch 1, wobei:
ein proximales Ende des Wirbelsäulenkatheters (15) einen Katheterhub (39) trägt; und
ein hinteres Ende der Stütznadel (19) einen Stützhub (35) trägt, der eine Trennungsstruktur (48B), (52C) oder (52D) aufweist, die konfiguriert ist, um den Katheterhub (39) vom Stützhub (35) zu trennen.

10. Wirbelsäulenkatheteranordnung (10) nach Anspruch 1, wobei der Wirbelsäulenkatheter (15) eine Leitung umfasst, die aus dem Kunststoffmaterial gebildet ist und an einem distalen Ende radial durch ein zweites Material verstärkt ist, um ein Abziehen von der Stütznadel (19) zu verhindern.

11. Wirbelsäulenkatheteranordnung (10) nach Anspruch 10, wobei das zweite Material aus einer Gruppe bestehend aus rostfreiem Stahldraht und einer Bandfeder (45) ausgewählt ist.

12. Wirbelsäulenkatheteranordnung (10) nach Anspruch 1, wobei der Wirbelsäulenkatheter (15) eine kraftabsorbierende Struktur umfasst, um ein Abknicken zu verhindern, wenn der Wirbelsäulenkatheter (15) gebogen wird.

13. Wirbelsäulenkatheteranordnung (10) nach Anspruch 12, wobei die kraftabsorbierende Struktur eine Bandfeder (45) umfasst.

14. Wirbelsäulenkatheteranordnung (10) nach Anspruch 12, wobei die kraftabsorbierende Struktur eine Knickschutztülle (18) umfasst, die auf einem Abschnitt davon angeordnet ist.

15. Wirbelsäulenkatheteranordnung (10) nach Anspruch 1, die ferner ein Mittelstylet (17) umfasst, das verschiebbar in der Stütznadel (19) angebracht ist, um das Eintreten von Materie durch die Öffnung (28) in der Nähe des ersten Endes (33) zu verhindern.

16. Wirbelsäulenkatheteranordnung (10) nach Anspruch 1, wobei die Stütznadel (19) konfiguriert ist, um während des Einführens der Wirbelsäulenkatheteranordnung (10) in den Patienten einer relativen Bewegung zwischen der Spitze (29) des Wirbelsäulenkatheters (15) und der nicht schneidenden Einstechspitze (22) entgegenzuwirken.

17. Wirbelsäulenkatheteranordnung nach Anspruch 7, wobei die Verbindung des Katheterhubs (39) mit der medizinischen Fluidtransferausrüstung eine LUER-LOCK-7-Verbindung ist.

## Revendications

1. Ensemble de cathéter rachidien (10) pour insertion à travers la dure-mère dans la colonne vertébrale d'un patient, ledit ensemble de cathéter rachidien (10) comprenant :
une aiguille de support (19) comportant une première extrémité (33) définissant une pointe de perçage non coupante (22), ladite aiguille de support (19) comprenant en outre un alésage creux muni d'un orifice (28) à proximité de ladite première extrémité (33) permettant l'accès audit alésage ; et
un cathéter rachidien (15) formé d'un matériau plastique monté de manière coulissante sur une partie de ladite aiguille de support (19), l'aiguille de support (19) étant adaptée pour s'étirer à travers un alésage du cathéter rachidien (15), de telle sorte que cette première extrémité (33) de ladite aiguille de support (19) fait saillie sur ledit cathéter rachidien (15), exposant ladite pointe de perçage non coupante (22) et ledit orifice (28), dans laquelle un bout (29) dudit cathéter rachidien (15) est effilé en courbure afin de pénétrer sans heurts à l'intérieur de la surface de l'aiguille de support (19) lorsque l'aiguille de support (19) est placée à l'intérieur du cathéter rachidien (15).

2. Ensemble de cathéter rachidien (10) selon la revendication 1, dans lequel ladite pointe de perçage non coupante (22) comprend un bout à pointe crayon (27).

3. Ensemble de cathéter rachidien (10) selon la revendication 1, dans lequel ledit ensemble de cathéter (10) comporte un bout (27) configuré et disposé de façon à fournir un signal de rétroaction pour indiquer une ponction durale.

4. Ensemble de cathéter rachidien (10) selon la revendication 1, dans lequel :
une extrémité postérieure de ladite aiguille de support (19) comporte un moyeu de support (35) possédant une première structure de fixation (34) ; et
une extrémité proximale dudit cathéter rachidien (15) comporte un moyeu de cathéter (39) possédant une seconde structure de fixation (37) configurée pour se fixer de manière amovible à la première structure de fixation (34) portée par ledit moyeu de support (35).

5. Ensemble de cathéter rachidien (10) selon la revendication 4, dans lequel les première et seconde structures de fixation (34) et (37) comprennent une connexion de type LUER-LOCK7.

6. Ensemble de cathéter rachidien (10) selon la revendication 4, dans lequel ledit moyeu de cathéter (39) est configuré pour une fixation en grande partie invisible sur la peau d'un patient par l'intermédiaire d'un élément adhésif intermédiaire.

7. Ensemble de cathéter rachidien (10) selon la revendication 4, dans lequel ledit moyeu de cathéter (39) est configuré pour une fixation à un équipement de transfert de fluides médicaux par une structure de fixation (110) afin de former une connexion globalement perpendiculaire à une direction d'insertion de cathéter.

8. Ensemble de cathéter rachidien (10) selon la revendication 1, dans lequel :
une extrémité postérieure de ladite aiguille de support (19) comporte un moyeu de support (35) ; et
une extrémité proximale dudit cathéter rachidien (15) comporte un moyeu de cathéter (39) possédant une structure de détachement (48B), (52C), ou (52D) configurée pour détacher le moyeu de cathéter (39) du moyeu de support (35).

9. Ensemble de cathéter rachidien (10) selon la revendication 1, dans lequel :
une extrémité proximale dudit cathéter rachidien (15) comporte un moyeu de cathéter (39) ;
et
une extrémité postérieure de ladite aiguille de support (19) comporte un moyeu de support (35) possédant une structure de détachement (48B), (52C), ou (52D) configurée pour détacher le moyeu de cathéter (39) du moyeu de support (35).

10. Ensemble de cathéter rachidien (10) selon la revendication 1, dans lequel ledit cathéter rachidien (15) comprend un conduit formé du matériau plastique et renforcé radialement à une extrémité distale par un second matériau afin d'éviter qu'il ne se décolle de l'aiguille de support (19).

11. Ensemble de cathéter rachidien (10) selon la revendication 10, dans lequel ledit second matériau est choisi dans le groupe comprenant un fil d'acier inoxydable et un ressort à ruban (45).

12. Ensemble de cathéter rachidien (10) selon la revendication 1, dans lequel ledit cathéter rachidien (15) comprend une structure d'absorption de force pour empêcher un étranglement lorsque le cathéter rachidien (15) s'incurve.

13. Ensemble de cathéter rachidien (10) selon la revendication 12, dans lequel ladite structure d'absorption de force comprend un ressort à ruban (45).

14. Ensemble de cathéter rachidien (10) selon la revendication 12, dans lequel ladite structure d'absorption de force comprend un manchon coudé (18) disposé sur une partie de celle-ci.

15. Ensemble de cathéter rachidien (10) selon la revendication 1, comprenant en outre un stylet central (17) monté de manière coulissante dans ladite aiguille de support (19) pour empêcher la pénétration de substances par ledit orifice (28) à proximité de ladite première extrémité (33).

16. Ensemble de cathéter rachidien (10) selon la revendication 1, dans lequel ladite aiguille de support (19) est configurée pour résister à un mouvement relatif entre ledit bout (29) dudit cathéter rachidien (15) et ladite pointe de perçage non coupante (22) pendant l'insertion dudit ensemble de cathéter rachidien (10) dans le patient.

17. Ensemble de cathéter rachidien selon la revendication 7, dans lequel la connexion dudit moyeu de cathéter (39) audit équipement de transfert de fluides médicaux est une connexion de type LUER-LOCK7.
